# EUROPÄISCHE PATENTSCHRIFT

(11) **EP 1 139 938 B1**
(45) Veröffentlichungstag und Bekanntmachung des Hinweises auf die Patenterteilung: **09.06.2004**
(21) Anmeldenummer: 99973469.2
(22) Anmeldetag: 05.11.1999
(51) Int. Cl.: A61F 2/46, A61B 17/16, A61B 17/17

(54) **Chirurgisches Gerätesatz zum mechanischen entfernen von Knochenzement**
Kit of chirurgical instruments for mechanically removing bone cement
Equipement chirurgical pour eliminer mecaniquement du ciment osseux

(30) Priorität: 22.12.1998 DE 19859412
(43) Veröffentlichungstag der Anmeldung: 10.10.2001
(73) Patentinhaber: Ferton Holding SA, 2800 Delémont (CH)
(72) Erfinder: SCHMIDT, Joachim, D-51427 Bergisch Gladbach (DE); RAABE, Thorsten, D-88682 Salem-Buggensegel (DE); MERKLE, Wolfgang, D-88709 Meersburg (DE)
(74) Vertreter: Dallmeyer, Georg, Dipl.-Ing.
(86) Internationale Anmeldenummer: PCT/EP1999/008492
(87) Internationale Veröffentlichungsnummer: WO 2000/036999

(56) Entgegenhaltungen:
- EP-A- 0 143 847
- EP-A- 0 379 785
- EP-A- 0 555 004
- WO-A-90/03769
- WO-A-93/01773
- CH-A- 566 127
- US-A- 4 337 773

## Beschreibung

Die Erfindung betrifft einen chirurgischen Gerätesatz zum mechanischen Entfernen von Knochenzement nach dem Oberbegriff des Anspruchs 1.

Derartige chirurgische Instrumente werden bei orthopädischen Operationen benötigt, um z.B. nach dem Entfernen einer Endoprothese (Hüfte, Knie, Schulter) den verbliebenen Knochenzement, z.B. aus PMMA zu entfernen, und einen einwandfreien Sitz der neu einzusetzenden Endoprothese zu ermöglichen.

Zum Entfernen des Zementes werden beispielsweise Meißelwerkzeuge mit Hilfe eines Hammers in den Zement hineingetrieben, um diesen von dem Knochen in der Knochenmarkhöhle abzuspalten.

Die Entfernung von Knochenzement ist vorzugsweise in der orthopädischen Chirurgie beim Wechsel oder Ausbaus von gelockerten oder infizierten Endoprothesen notwendig.

Die Zahl der Prothesenwechsel nimmt insbesondere in der Hüftendoprothetik stetig zu. Die Entfernung des Knochenzementes bei zementfixierten Prothesen ist mühsam und zeitaufwendig. Bisher wird die Zemententfernung in der Regel mit verschieden geformten Meißeln durchgeführt, deren sichere Anwendung z.B. in der schlecht einsehbaren Tiefe der Markhöhle problematisch ist. Es können neben dem hohen Zeitaufwand Knochenschädigungen resultieren, die die Neuimplantation einer Prothese unmöglich machen oder aber zu einem übergroßen Knochenverlust führen.

Extraktionsinstrumente werden entweder ohne direkte Sichtkontrolle gesetzt oder die Position muß mittels Röntgenbildverstärker kontrolliert werden. Fehlpositionierungen sind möglich und auch unter Röntgenbildverstärkerkontrolle nicht vollständig zu vermeiden. Fehlpositionierungen führen zu Knochenschädigungen, die Operationserweiterungen notwendig machen.

Der Einsatz des Röntgenbildverstärkers stellt eine Strahlenbelastung für den Patienten und das OP-Personal dar. Bekannte Extraktionsinstrumente, im speziellen Bohrer, führen aufgrund der Bauweise bei Penetration des Zementes oder Markraumstoppers in Peripherrichtung zu einer zwangsläufigen Druckerhöhung in der Markraumhöhle, die eine Fettembolisation auslösen kann.

Bei einer Hüftgelenkswechseloperation muß der zur Fixierung der alten Prothese eingesetzte Zement vor dem erneuten Einbringen der Revisionsprothese entfernt werden. Da es sich bei dem Oberschenkelknochen um einen langen Röhrenknochen handelt, ist der Zugang zu dem distalen Zementrest und dem Markraumstopper durch die Öffnung am proximalen Ende schwierig.

Während der Entfernung des Zementes bzw. Markraumstoppers kommt es mit herkömmlichen Werkzeugen zu einem Anstieg des Druckes im Knochen unterhalb des Markraumstoppers. Beim Arbeiten am distalen Zementrest bzw. Markraumstopper wird eine Kraft auf diese ausgeübt. Dadurch kann der dahinter liegende Raum komprimiert werden - ähnlich wie bei einer Luftpumpe - und eine Druckerhöhung bewirken, welche das dort reichlich vorhandene Markraumfett in die Blutbahn drücken kann. Dies führt zu Fettembolien, die häufig bleibende Schäden beim Patienten hinterlassen und nicht selten sogar zum Tod führen. Untersuchungen haben gezeigt, daß eine Fettembolie unter Umständen nicht anaesthetisch erfaßt wird. Der Tod tritt plötzlich und unvorhergesehen ein. Besonders kritisch sind Fettembolien, wenn eine Sepsis vorliegt, da dann zusammen mit dem Fett auch Keime in die Blutbahn gedrückt werden und die Sepsis verschleppt wird.

Die WO-A-93/01773 betrifft eine Vorrichtung zum Entfernen einer Hüftprothese, bei der in einem ersten Schritt eine Pilotbohrung mit einem Bohrwerkzeug ausgeführt wird. In diese Bohrung wird dann ein Führungsdraht eingesetzt.

Der Führungsdraht dient dann dazu, mehrere Reibahlen unterschiedlichen Durchmessers zu führen, die den alten Zement entfernen. Somit ist weder das erstmalig benutzte Bohrwerkzeug noch das Extraktionswerkzeug zur Aufnahme eines Zentrierstiftes mit einer Bohrung versehen.

Der Erfindung liegt demzufolge die Aufgabe zugrunde, einen chirurgischen Gerätesatz zum Entfernen von distalen Zementresten und Markraumstoppern zu schaffen, mit dem Fehlpositionierungen des Extraktionswerkzeuges und Druckerhöhungen im Markraum vermieden werden können.

Die Erfindung sieht in vorteilhafter Weise vor, daß das Bohrwerkzeug und das Extraktionswerkzeug zur Aufnahme eines Zentrierstiftes eine zu dem jeweiligen Werkzeugschaft koaxiale Bohrung aufweisen, daß der Bohrungsdurchmesser der koaxialen Bohrungen des Bohrwerkzeuges und des Extraktionswerkzeugs dem Durchmesser des Zentrierstiftes im wesentlichen angepaßt ist, und daß der zuvor an einer vorbestimmten Stelle in dem Zement oder Markraumstopper und in dem distal darunterliegenden Markraum verankerte Zentrierstift als axiale Führung für das Bohrwerkzeug bzw. das Extraktionswerkzeug dient.

Ein wesentliches Merkmal dieses Extraktionswerkzeuges ist es, daß zumindest das distale Ende des Extraktionswerkzeuges zentrisch kanüliert ist. Bei der Zemententfernung wird zunächst ein Zentrierstift in den distalen Zementrest bzw. Markraumstopper eingedreht. Mit einem zumindest am distalen Ende kanülierten Bohrer wird dann der Zementrest bzw. der Markraumstopper vollständig durchbohrt, wobei der Zentrierstift als axiale Führung dient. Nach dem Vorbohren wird das Extraktionswerkzeug in die zuvor hergestellte Bohrung bis zum distalen Ende des Zementrestes bzw. Markraumstoppers eingedreht, wobei der verbliebene Zentrierstift wieder als Führung dient. Anschließend zieht der Anwender den distalen Zementrest bzw. den Markraumstopper heraus. Sitzt dieser fest im Knochenzement bzw. Markraumstopper, kann er mit einem speziellen Hammer gegen eine Ansatzfläche des Extraktionswerkzeuges schlagen und somit das Extraktionswerkzeug mit dem Zementstopfen oder Markraumstopper ausschlagen.

Die kanülierte Ausführung des Extraktionswerkzeugs besitzt folgende Vorteile:

Durch die Führung des Zentrierstiftes ist eine exakte Positionierung der Instumentenspitze trotz des erschwerten Zugangs in dem langen Röhrenknochen und der schlechten Sichtverhältnisse möglich. Damit wird zudem verhindert, daß sowohl beim Vorbohren als auch beim Eindrehen des Extraktionswerkzeuges der Knochen aufgrund eines Auswanderns des Extraktionswerkzeuges perforiert wird oder reißt (Fissur). Eine Knochenperforation oder Fissur führt zu verlangsamter Heilung und kann im schlimmsten Fall die Implantation der Revisionsprothese unmöglich machen.

Die koaxialen Bohrungen sind proximal offen und bilden damit einen Druckausgleichskanal.

Mit dem kanülierten Bohrwerkzeug und dem kanülierten Extraktionswerkzeug findet vor und während der Entfernung des Zementrestes bzw. des Markraumstoppers durch die axiale Bohrung in den Werkzeugen ein Druckausgleich statt, der einen Druckanstieg im Markraum unterhalb des Zementrestes bzw. des Markraumstoppers. verhindert, so daß die Gefahr einer Fettembolie oder einer verschleppten Sepsis reduziert wird.

Vorzugsweise weist das Extraktionswerkzeug ein selbstschneidendes Außengewinde auf, das sich in die von dem Bohrwerkzeug hergestellte Bohrung mit geringem Kraftaufwand eindrehen läßt.

Das Außengewinde ist vorzugsweise in der Art einer Kortikalisschraube geformt, so daß das Außengewinde in der vorgebohrten Bohrung aufgrund einer Selbsthemmung verankert wird. Die koaxialen Bohrungen in dem Extraktionswerkzeug und dem Bohrwerkzeug für die Aufnahme des Zentrierstifts weisen einen Durchmesser im Bereich von ca. 1,5 bis 4 mm, vorzugsweise ca. 2-3 mm, auf.

Der Zentrierstift weist einen im Verhältnis zu den koaxialen Bohrungen des Bohrwerkzeugs und des Extraktionswerkzeuges geringeren Durchmesser auf. Damit ist es möglich, daß sowohl das Bohrwerkzeug als auch das Extraktionswerkzeug von dem gleichen Zentrierstift mit Spiel geführt werden, so daß eine exakte Positionierung der Werkzeuge möglich ist.

Die distalen Enden der koaxialen Bohrungen des Bohrwerkzeuges und des Extraktionswerkzeuges können nach außen konisch erweitert sein. Die konische Erweiterung des distalen Endes der Bohrungen erleichtert das Einführen der Werkzeuge.

Die koaxialen Bohrungen in dem Bohrwerkzeug bzw. in dem Extraktionswerkzeug können einen Anschlag für die maximale Eindringtiefe des Werkzeuges aufweisen. Auf diese Weise kann verhindert werden, daß die Werkzeuge zu tief in den distalen Markraum bzw. in den Zementrest oder Markraumstopper hineingedreht werden.

Im folgenden wird unter Bezugnahme auf die Zeichnungen ein Ausführungsbeispiel der Erfindung näher erläutert:

Es zeigen:
- Fig. 1: einen Querschnitt durch das proximale Ende eines Oberschenkelknochens nach entfernter Hüftgelenksprothese,
- Fig. 2: die Einbringung des Zentrierstifts,
- Fig. 3: die Führung des Bohrwerkzeuges mit dem Zentrierstift,
- Fig. 4: die Führung des Extraktionswerkzeugs mit dem Zentrierstift,

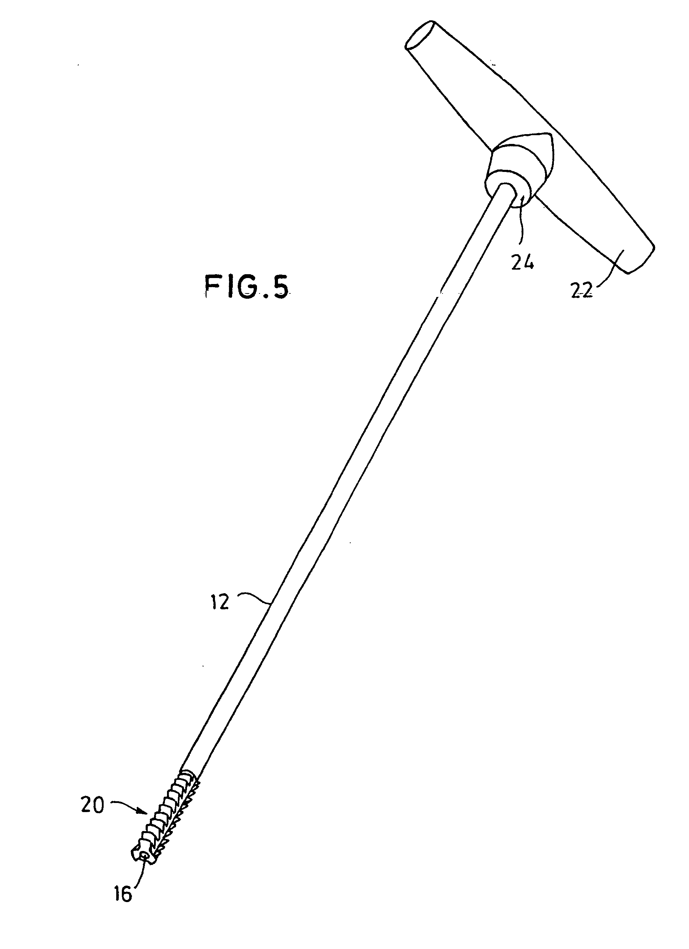

- Fig. 5: eine perspektivische Ansicht des Extraktionswerkzeugs,
- Fig. 6: eine Seitenansicht des Extraktionswerkzeugs, und
- Fig. 7: eine Ansicht des Bohrwerkzeugs gemäß Linie VII-VII in Fig. 6.

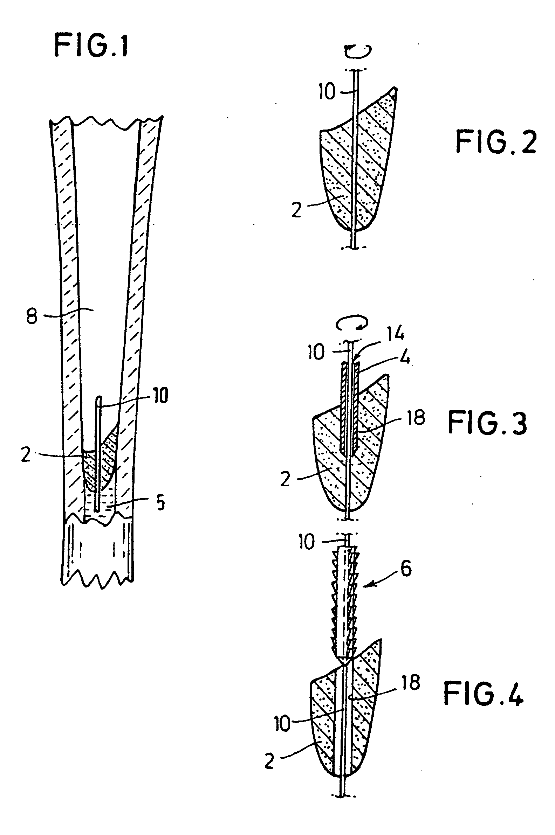

Fig. 1 zeigt einen Querschnitt durch das proximale Ende eines Oberschenkelknochens, bei dem eine Hüftgelenksprothese entfernt worden ist. Die proximalen peripheren Zementreste 2 können in herkömmlicher Weise mit Meißeln entfernt werden. Dagegen muß die Zemententfernung bzw. die Entfernung eines Markraumstoppers am distalen Ende der Markraumhöhle 8 mit einem Extraktionswerkzeug 6 erfolgen. Ein Markraumstopper aus Zement oder Knochenmaterial wird beim Einsatz der Prothese eingesetzt, um die Markraumhöhle distal gegen den darunterliegenden Markraum 5 zu verschließen.

Fig. 2 zeigt die Einbringung ein Zentrierstiftes 10 durch den Zementrest 2 am distalen Ende der Markraumhöhle 8 in den Markraum 5.

Unter endoskopischer oder direkter Sicht wird zunächst der Zentrierstift 10 an der gewünschten Stelle im Zementrest 2 oder im Markraumstopper positioniert. Auf einer Schrägfläche kann es dabei notwendig sein, den gewünschten Eintrittspunkt in geeigneter Weise mit einem Körnwerkzeug vorzukörnen. Der Zentrierstift 10 kann mit einer Bohrspitze versehen sein, so daß er durch Drehung leicht durch den Zementrest 2 in den distal unter dem Zementrest 2 befindlichen Markraum 5 eindrehbar ist.

Der Zentrierstift weist einen Durchmesser von ca. 1,5 bis 4 mm, vorzugsweise 2,5 mm, auf. Er kann auch mit einem Außengewinde versehen sein, um die Verankerung des Zentrierstifts 10 in dem Zementrest 2 und dem Markraum 5 zu verbessern.

Nachdem der Zentrierstift 10 in dem Zementrest 2 bzw. einem Markraumstopper und dem Markraum 5 derart fixiert ist, daß ein Teil des Zentrierstiftes 10, wie aus Fig. 1 ersichtlich proximal über den Zementrest 2 übersteht, kann er als Führungsmittel für ein Bohrwerkzeug 4 und ein Extraktionswerkzeug 6 dienen.

Fig. 3 zeigt das distale Ende eines kanülierten Bohrwerkzeuges 4, das mit Hilfe des Zentrierstiftes 10 exakt positioniert werden kann, ohne daß eine Gefahr des Abrutschens des Bohrwerkzeuges bestehen kann. Das Bohrwerkzeug 4 ist mit einer zu seiner Längsachse koaxialen Bohrung 14 versehen, die dem Durchmesser des Zentrierstiftes 10 angepaßt ist, so daß das Bohrwerkzeug 4 mit Spiel auf den Zentrierstift 10 aufgesetzt werden kann und durch den Zementrest 2 hindurchgebohrt werden kann.

Die koaxiale Bohrung 14 des Bohrwerkzeuges 4 ist zum proximalen Ende hin offen, um eine Druckentlastung zu ermöglichen.

Die Bohrung 14 kann auch einen Anschlag für den Zentrierstift 10, z.B. durch Bildung eines Absatzes aufweisen, um die Eindrehtiefe des Bohrwerkzeuges 4 zu begrenzen.

Nach Herstellung der Bohrung 18 mit dem Bohrwerkzeug 4 durch den Zementrest 2 bzw. Markraumstopper hindurch kann das Extraktionswerkzeug 6 auf den Zentrierstift 10 aufgesetzt werden, und mit Hilfe der Führung des Zentrierstiftes 10 in die Bohrung 18 bis zum distalen Ende des Zementrestes 2 bzw. Markraumstoppers eingeschraubt werden. Der Bohrungsdurchmesser der Bohrung 18 entspricht im wesentlichen dem Kerndurchmesser eines Außengewindes 20 am distalen Ende des Extraktionswerkzeuges 6.

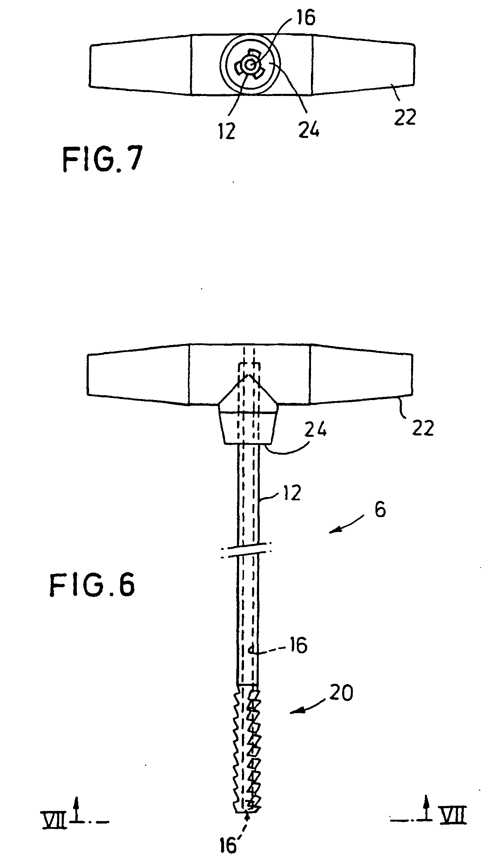

Das Extraktionswerkzeug 6 weist wie das Bohrwerkzeug 4 in seinem Schaft 12 eine zu seiner Längsachse koaxiale Bohrung 16 auf, deren Durchmesser ebenfalls an den Durchmesser des Zentrierstiftes 10 angepaßt ist, so daß sich das Extraktionswerkzeug 6 mit Spiel um den Zentrierstift 10 drehen läßt. Das Extraktionswerkzeug 6 wird dann in der Bohrung 18 aufgrund des Außengewindes 20 in der Art einer Selbsthemmung verankert. Der Schaft des Extraktionswerkzeuges 6 weist eine Länge von beispielsweise 300 mm auf. Das Außengewinde 20 ist in der Art einer Kortikalisschraube gestaltet. Wie am besten aus den Fign. 6 und 7 ersichtlich, ist dieses Außengewinde jeweils um 120 ° versetzt freigeschnitten. Die koaxiale Bohrung 16 ist vorzugsweise vom distalen bis zum proximalen Ende durchgängig und verläuft auch durch einen an dem proximalen Ende vorgesehenen Griff 22. Am unteren Teil des Griffes ist eine Aufschlagfläche 24 vorgesehen, die so ausgebildet ist, daß mit in der Orthopädie üblichen Gabelhämmern das Extraktionswerkzeug 6 mit dem distalen Zementrest 2 bzw. dem Markraumstopper ausgeschlagen werden kann.

Auch die koaxiale Bohrung 16 kann mit einem Absatz oder Ringbund als Anschlagfläche für den Zentrierstift 10 versehen sein, um die Eindrehtiefe des Extraktionswerkzeuges 6 zu begrenzen.

Falls die koaxiale Bohrung 16 nur in einem distalen Abschnitt des Schaftes 12 verläuft, ist diese durch einen radialen Kanal durch den Schaft 12 nach außen verbunden, um einen Druckausgleich zu ermöglichen.

## Patentansprüche

1. Chirurgischer Gerätesatz zum Entfernen von distalem und /oder peripherem Zement (2) und/oder einem Markraumstopper in einer Knochenmarkhöhle (8) beim Ersatz von Endoprothesen, mit einem Bohrwerkzeug (4) und einem Zentrierstift (6),
**dadurch gekennzeichnet, daß** er weiter ein Extraktionswerkzeug aufweist, und
daß das Bohrwerkzeug (4) und das Extraktionswerkzeug (6) zur Aufnahme des Zentrierstiftes (10) eine zu dem jeweiligen Werkzeugschaft (12) koaxiale Bohrung (14,16) aufweisen,
daß der Bohrungsdurchmesser der koaxialen Bohrungen (14,16) des Bohrwerkzeuges (4) und des Extraktionswerkzeugs (6) dem Durchmesser des Zentrierstiftes (10) im wesentlichen angepaßt ist, und
daß der zuvor an einer vorbestimmten Stelle in dem Zement (2) oder Markraumstopper und in dem distal darunter liegenden Markraum (5) verankerte Zentrierstift (10) als axiale Führung für das Bohrwerkzeug (4) oder das Extraktionswerkzeug (6) dient.

2. Chirurgischer Gerätesatz nach Anspruch 1, **dadurch gekennzeichnet, daß** die koaxialen Bohrungen (14,16) des Bohrwerkzeuges (4) und des Extraktionswerkzeuges (6) proximal offen sind und damit einen Druckausgleichskanal bilden.

3. Chirurgischer Gerätesatz nach Anspruch 1 oder 2, **dadurch gekennzeichnet, daß** das Extraktionswerkzeug (6) ein selbstschneidendes Außengewinde (20) aufweist.

4. Chirurgischer Gerätesatz nach Anspruch 3, **dadurch gekennzeichnet, daß** das Außengewinde (20) in der Art einer Kortikalisschraube geformt ist.

5. Chirurgischer Gerätesatz nach einem der Ansprüche 1 bis 4, **dadurch gekennzeichnet, daß** die koaxialen Bohrungen (14,16) für die Aufnahme des Zentrierstifts (10) einen Durchmesser im Bereich von 1,5 bis 4 mm, vorzugsweise ca. 2 bis 3 mm, aufweisen.

6. Chirurgischer Gerätesatz nach einem der Ansprüche 1 bis 5, **dadurch gekennzeichnet, daß** der Zentrierstift (10) einen im Verhältnis zu den Bohrungen (14,16) des Bohrwerkzeuges (4) und des Extraktionswerkzeuges (6) geringeren Durchmesser aufweist.

7. Chirurgischer Gerätesatz nach einem der Ansprüche 1 bis 6, **dadurch gekennzeichnet, daß** die distalen Enden der Bohrungen (14,16) des Bohrwerkzeuges (4) und des Extraktionswerkzeuges (6) nach außen konisch erweitert sind.

8. Chirurgischer Gerätesatz nach einem der Ansprüche 1 bis 7, **dadurch gekennzeichnet, daß** die koaxialen Bohrungen (14,16) in dem Bohrwerkzeug (4) bzw. in dem Extraktionswerkzeug einen Anschlag für den Zentrierstift (10) aufweisen, um die maximale Eindrehtiefe zu begrenzen.

9. Chirurgischer Gerätesatz nach einem der Ansprüche 1 bis 8, **dadurch gekennzeichnet, daß** das Extrationswerkzeug (6) ein Griffteil (22) aufweist, und daß an der Unterseite des Griffteils (22) eine Aufschlagfläche (24) für einen Gabelhammer angeordnet ist.

## Claims

1. Surgical instrument kit for removing distal and/or peripheral cement (2) and/or a medullary block in a medullary cavity (8) when endoprostheses are replaced, said surgical instrument kit comprising a drilling tool (4) and a centering bolt (6),
**characterized in that**
the surgical instrument kit further comprises an extraction tool, and the drilling, tool (4) and the extraction tool (6) comprise a bore (14,16) extending coaxially to the respective tool shaft (12) for receiving a centering bolt (10),
the bore diameter of the coaxial bores (14,16) of the drilling, tool (4) and the extraction tool (6) is substantially adapted to the diameter of the centering bolt (10), and
the centering bolt (10) anchored at a predetermined location in the cement (2) or the medullary block and in the medullary space (5) located distally below the cement or the medullary block is configured as an axial guide for the drilling tool (4) or the extraction tool (6).

2. Surgical instrument kit according to claim 1, **characterized in that** the coaxial bores (14,16) of the drilling tool (4) and the extraction tool (6) are open at the proximal end thus forming a pressure compensation duct.

3. Surgical instrument kit according to claim 1 or 2, **characterized in that** the extraction tool (6) comprises a self-cutting male thread (20).

4. Surgical instrument kit according to claim 3, **characterized in that** the male thread (20) is configured as a cortical screw.

5. Surgical instrument kit according to one of claims 1 to 4, **characterized in that** the coaxial bores (14,16) for receiving the centering bolt (10) have a diameter ranging from 1.5 to 4 mm, preferably from approximately 2 to 3 mm.

6. Surgical instrument kit according to one of claims 1 to 5, **characterized in that** the centering bolt (10) has a smaller diameter with respect to the bores (14,16) of the drilling tool (4) and the extraction tool (6).

7. Surgical instrument kit according to one of claims 1 to 6, **characterized in that** the distal ends of the bores (14,16) of the drilling tool (4) and the extraction tool (6) are flared conically, towards the outside.

8. Surgical instrument kit according to one of claims 1 to 7, **characterized in that** the coaxial bores (14,16) in the drilling tool (4) and the extraction tool, respectively, comprise a stop for the centering bolt (10) to define the maximum penetration depth.

9. Surgical instrument kit according to one of claims 1 to 8, **characterized in that** the extraction tool (6) comprises a grip portion (22), and that on the bottom side of the grip portion (22) an impact surface (24) for a forked hammer is arranged.

## Revendications

1. Equipement chirurgical pour éliminer du ciment distal et/ou périphérique (2) et/ou un bouchon dans une cavité médullaire d'un os (8) lors du remplacement de prothèses d'extrémité, comprenant un outil de perçage (4) et une tige de centrage (10),
**caractérisé en ce qu'**il comprend en outre un outil d'extraction, et **en ce que** l'outil de perçage (4) et l'outil d'extraction (6) comportent un perçage (14, 16), coaxial à la tige d'outil correspondante (12), afin de recevoir la tige de centrage (10),
**en ce que** le diamètre des perçages coaxiaux (14, 16) de l'outil de perçage (4) et de l'outil d'extraction (6) est essentiellement adapté au diamètre de la tige de centrage (10), et
**en ce que** la tige de centrage (10), ancrée auparavant à un endroit prédéterminé dans le ciment (2) ou le bouchon de cavité médullaire et dans la cavité médullaire (5) sous-jacente du côté distal, sert de guide axial pour l'outil de perçage (4) ou bien l'outil d'extraction (6).

2. Equipement chirurgical selon la revendication 1, **caractérisé en ce que** les perçages coaxiaux (14, 16) de l'outil de perçage (4) et de l'outil d'extraction (6) sont ouverts du côté proximal et forment ainsi un canal de compensation de pression.

3. Equipement chirurgical selon la revendication 1 ou 2, **caractérisé en ce que** l'outil d'extraction (6) comporte un filetage extérieur (20) auto-taraudant.

4. Equipement chirurgical selon la revendication 3, **caractérisé en ce que** le filetage extérieur (20) est formé à la manière d'une vis corticale.

5. Equipement chirurgical selon l'une des revendications 1 à 4, **caractérisé en ce que** les perçages coaxiaux (24, 16) pour recevoir la tige de centrage (10) ont un diamètre de l'ordre de 1,5 mm à 4 mm, avantageusement de 2 à 3 mm environ.

6. Equipement chirurgical selon l'une des revendications, 1 à 5, **caractérisé en ce que** la tige de centrage (10) a un diamètre qui est inférieur à celui des perçages (14, 16) ménagés dans l'outil de perçage (4), et dans l'outil d'extraction (6).

7. Equipement chirurgical selon l'une des revendications 1 à 6, **caractérisé en ce que** les extrémités distales des perçages (14, 16) de l'outil de perçage (4) et de l'outil d'extraction (6) s'évasent vers l'extérieur en cône.

8. Equipement chirurgical selon l'une des revendications 1 à 7, **caractérisé en ce que** les perçages coaxiaux (14, 16) ménagés dans l'outil de perçage (4) ou dans l'outil d'extraction présentent une butée pour la tige de centrage (10) afin de limiter la profondeur de vissage maximale.

9. Equipement chirurgical selon l'une des revendications 1 à 8, **caractérisé en ce que** l'outil d'extraction (6) comporte une partie de préhension (22) et **en ce qu'**il est prévu du côté inférieur de la partie de préhension (22) une surface d'appui qui est destinée à un marteau bifurqué.
